# EUROPEAN PATENT APPLICATION

(11) **EP 2 392 922 A1**
(43) Date of publication of application: **07.12.2011**
(21) Application number: 10380077.7
(22) Date of filing: 08.06.2010
(51) Int. Cl.: G01N 33/18, G01N 1/10, G01N 1/14, G01N 1/16

(54) **Autonomous multi-parametric buoy for water sampling, monitoring, data collection, transmission, and analysis**

(30) Priority: 04.06.2010 ES 201030858
(71) Applicant: Ecofloat Galicia, S.L., 15940 Pobra do Caramiñal (A Coruña) (ES)
(72) Inventor: Barceló Serrano, José Ismael, 15940 Pobra de Caramiñal (A Coruña) (ES)
(74) Representative: Carvajal y Urquijo, Isabel

(57) **Abstract**

Beacon used to control water quality and warn about the presence of undesired components, and remotely and autonomously monitor environmental behaviour. It consists of a communication module and a sample intake and analysis module.

## Description

### FIELD OF THE INVENTION

The present invention refers to water control and analysis systems which include water collection and information management.

### STATE OF THE ART

Within the current state of the art, there exist fixed microbiologic analysis systems which require great room for their installation and imply high energy costs. Besides, they have a particular location without flexibility for their use in areas far away from the coast or in places where quality control is only needed in certain occasions.

### DESCRIPTION OF THE INVENTION

Many deficiencies have been found in the devices known in the state of the art. Among others, we can mention shipping needs, multiple work equipment, specific and discontinuous data and space limitations.

By applying the features mentioned in the claims, the present invention solves the aforementioned disadvantages.

To achieve that, the present invention presents an autonomous system featuring continuous remote communication. System flexibility is very important for the integration of new equipment so that more parameters can be measured. For this reason, several internal communication protocols have to be applied to implement communication from the main system to each one of the equipment units. In an autonomous system, energy management is another important issue to be considered, therefore, an equipment connection and disconnection system has been applied so that the equipment units work only when they are really operating.

By establishing a remote control system, the efficiency and reliability of the results is increased. This also allows greater reaction speed and cost reduction.

### BRIEF DESCRIPTION OF THE DRAWINGS

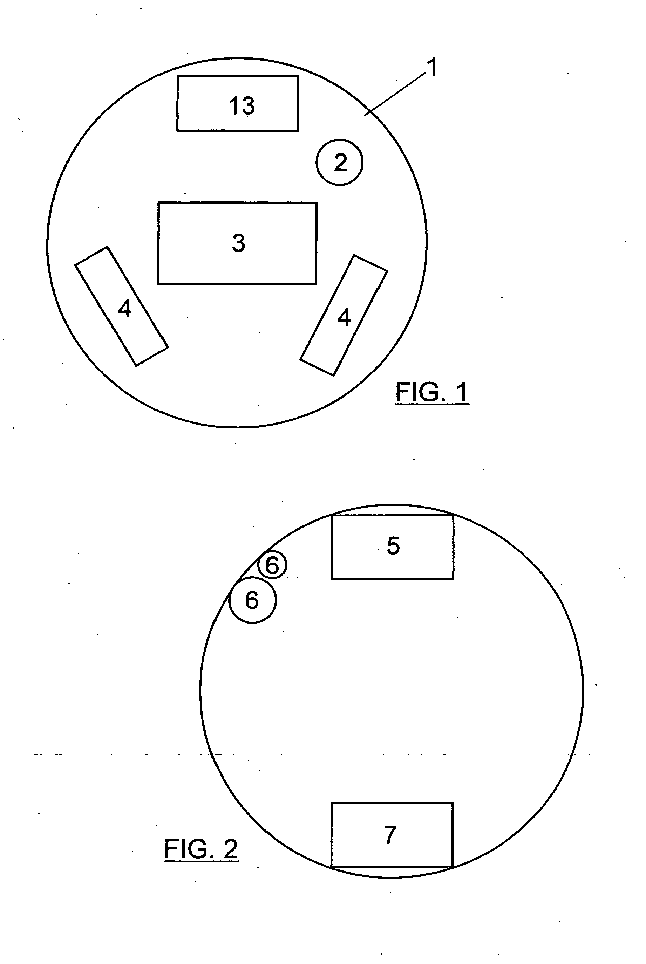

Fig 1 shows an embodiment of the invention according to a lower view, where the following can be seen:
A wave sensor (3), a battery (4), a disposal container or drum (1) to collect waste produced by a microbial sensor (5) not shown in figure 1. An Exit pipe FQ (2) allows contact with water through its interior. It is used to place sensors in continental, coastal and transition waters, these sensors being able to analyze physicochemical parameters found in water. As its upper end is above the buoy floating line, water does not reach the cylinder containing the devices.

Fig 2 shows an embodiment of the invention according to a medium height view, where the following can be seen:
The microbial sensor (5), some bottles (6) containing chemical products and the control system (7).

Fig 3 shows an example of a hydraulic circuit design, showing: filters (10, 24, 25, 26, 27, 28) for the inlet of air and several chemical components pumped by a peristaltic pump (22) and selectively mixed by a multiple valve (21) with several inlets and one outlet into a deposit (14). A water sample (23) is extracted by the pump (22) and its attached valve (23) and is analyzed in the deposit (14). In case there is an excess of liquid in the deposit, there is a second pump (12) designed to empty waste into a disposal drum (13).

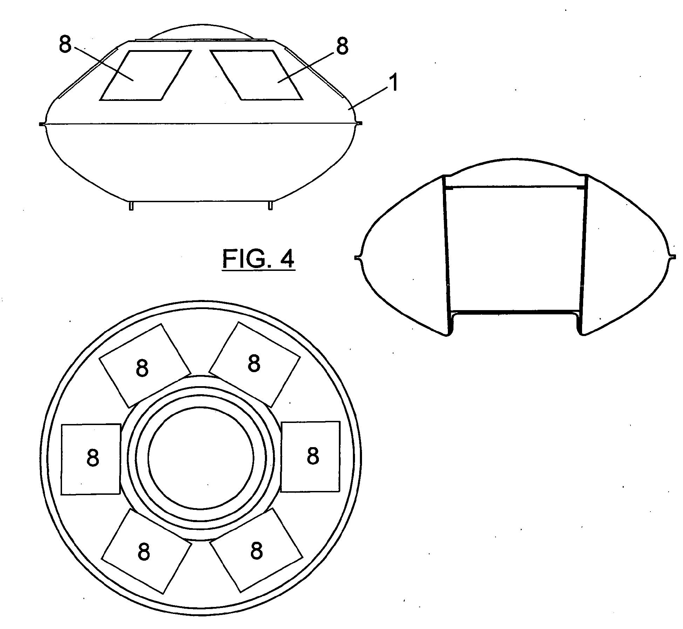

Fig 4 shows an example of the casing where solar panels (9) are located.

### DETAILED DESCRIPTION OF A PREFERRED EMBODIMENT

Preferably, the beacon is made of FRP (Fiberglass Reinforced Polyester). It is a composite material formed by a strong fiberglass structure. Fiberglass reinforcement provides the composite with mechanical strength, dimensional stability and heat resistance. The casing undergoes paint treatments which add chemical, dielectric and outdoor resistance.

In a preferred embodiment of the invention, the buoy frame consists of three well-defined pieces made of FRP, as shown in Fig 5.
- Two semicircular halves, which frame the structure as a casing and form a flat sphere, which has a 250cm diameter and is 135cm high approximately.
- A vertical cylinder which goes across both casings and which has 4 stainless steel chains in its lower end, these chains functioning as legs and come out from the beacon through its lower end to finally set anchor. Holes drilled in the lower part of the cylinder work as through-hull fittings and allow the passage of wires or pipes used in the equipment outdoors.
- A FRP flat cover which has 20 holes, through which it is coupled to 20 studs coming out from the upper part of the beacon. A hatch is located at the centre of the beacon.

Each beacon has 6 solar panels throughout the whole circumference. They are coupled to their respective boxes with the same measures as the panels and connected to the batteries by internal wiring.

This system has a main battery and it may also have an additional battery depending on the location where the analysis system is intended to be used.

### Equipment Arrangement inside the Beacon

Inside the beacon, the pieces of equipment are arranged in two levels. Some are fixed to the ground and others are fixed to the beacon wall as shown in Fig 1.

A disposal drum (13) is fixed to the ground and acts as a container to collect waste produced by the microbial sensor, a pipe housing the physicochemical sensor. (2) inside it, a wave sensor (3) located at the centre of the beacon and one or two batteries (4) depending on the installation.

The microbial sensor (5) is attached to the beacon walls, on top of the containers fixed to the ground, bottles (6) are fixed on the right along with the chemical products needed for the sensor (5) to operate, and the control system is also fixed to the beacon walls.

### Control System

it consists of a group of electronic components (7) which control communication between the different equipment installed in the beacon.

Besides, it has a modem allowing access to the network by means of wireless technologies such as GPRS or UMTS so that acquired data can be added in real time.

This system can be remotely set up and allows control of the data acquisition time in each sensor as well as the activation and deactivation thereof.

### Microbial Sensor

The sensor can quantify Escherichia coli and intestinal Enterococcus organisms. It can also quantify chlorophyll and blue-green algae.

The method used for analytical determination of Escherichia coli and Enterococcus is based on the use of culture media containing fluorogenic substrates. Said substrates are hydrolyzed by highly specific enzymes belonging to the enterococcus group (E. faecium and E. faecalis) and to the E. coli group, releasing fluorescent molecules into the medium.

Fluorescence is an emission process in which molecules are stimulated by absorption of ultra violet radiations. Under these stimuli species go back to their basic state, releasing excess of energy as photons.

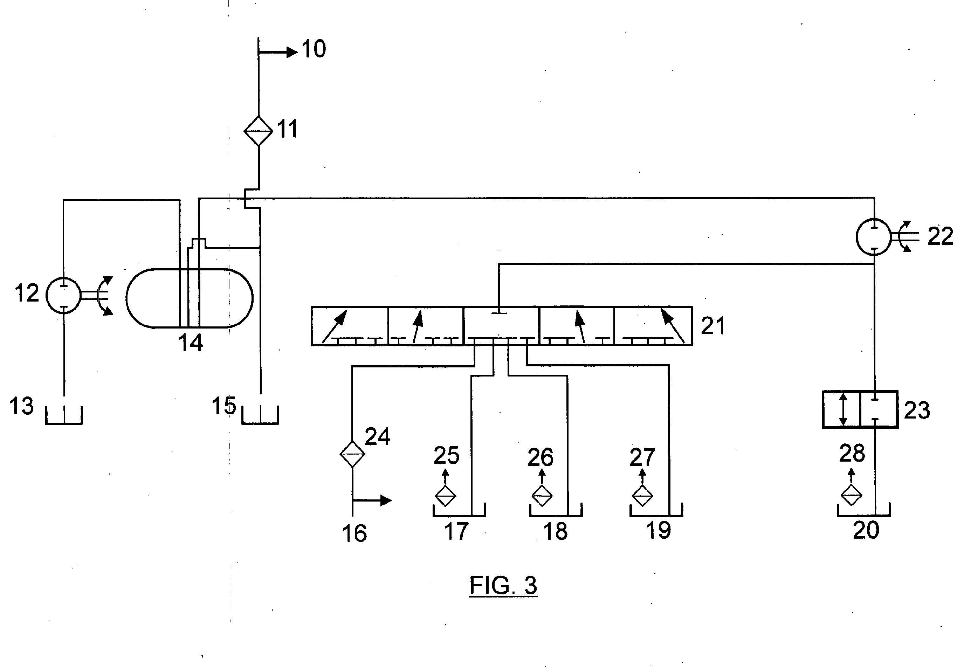

An example of a microbial sensor is shown in the hydrostatic diagram, in Fig 3. It consists of:
- Two peristaltic pumps (12, 22) which can rotate in both directions. The use of peristaltic pumps is important for our system to avoid contact between the fluid being pumped and the pump, instead, the fluid should always be in contact with the pipe. This will prevent both contamination and erosion-related issues caused by the chemical products we use.
- Five electrovalves. In the example shown, four of them (v1, v2, v3, v4) are integrated in a cross-like shape into a multi-valve device (21), with 4 inlets and 1 outlet. Each of these four valves controls the intake of air (16), caustic soda (25), distilled water (18) and reactive (19), respectively. Electrovalve v5 (23) controls the sample intake (20). Electrovalves and pump preferably work on 12Vcc.

- A deposit (14) for biological culture. The deposit has the following inlets and outlets:
- An inlet coming from the first pump (22), all fluids go into the deposit (14) through this intake. An outlet through which the second pump (12) operates and releases liquid from the deposit (14) into a disposal drum (13). Finally, it has a third connection to an overflow container (15). It is used as an evacuation line in case the deposit (14) exceeds its capacity. As it can be seen in the scheme (Fig 3), the evacuation line connects to the overflow container (15) downstream, and also to a previous filter (11) through the part above the atmosphere.

### Control and Acquisition System

The control system consists of two microcontrollers in charge of the control of the hydraulic circuit, optical system, heating system and analogical signal acquisition.

The first microcontroller controls five electrovalves, two peristaltic pumps (12, 22), overflow sensor and flowmeter. Thus, the different phases involved in the sample taking, washing and emptying processes are implemented. The combination of these actuators makes it possible to selectively introduce and dose liquid into the deposit (14) with the different liquids the system comprises. The deposit (14) is emptied in the same way.

The second microcontroller controls the optical system through the measuring and testing leds. This controller also monitors the temperature in the deposit by means of a feedback control loop, the actuator of which is a peltier. This microcontroller obtains analogical signals from the optical system: fluorescence, temperature and pressure in the deposit.

The different sensors allow evaluation of any anomalous operation of the sensor such as wrong dosage, hydraulic system stoppage or optical system failure.

Each device has its own communication protocol. Therefore, it is necessary to have a control system capable of communicating with each of them and which also has a connection with the outside through wireless technology.

In an embodiment, the control system also comprises:
- An embedded plate with a Windows CE 5.0 operating system or Debian GNU/Linux 5.0 (Lenny), according to the system requirements.
- An electronic system which manages and transforms different electric potentials and controls the activation and deactivation of the devices.
- A wireless communication system which may be through radio, GSM or GRPS.

For the integration of multiple devices and automation of the process of sample taking for analysis, data acquisition and result sending, several stages are defined: Configuration: The control system is set up so as to regularly gather data.

Acquisition: The control system detects connected and configured devices, communicates with them through the communication protocol and records gathered data.

Data sending: The control system can be configured to send gathered data at a certain pre-determined date and with certain frequency, or the system can gather data at any given time.

References:
1: casing
2: physicochemical sensor
3: wave sensor
4: battery
5: microbial sensor
6: bottles
7: electronic components
8: solar panel
9: solar panels
10: air inlet
11: filter
12: second pump
13: disposal container
14: analysis deposit or container
15: overflow container
16: air inlet
17:. caustic soda container
18: distilled water container
19: reactive container
20: sea water inlet
21: multiple valves with 4 inlets and one outlet
22: first pump
23: electrovalve for the sample
24 25 26 27: filter

## Claims

1. Water quality control beacon comprising a plurality of containers (14,17, 18, 19, 20), at least one pump (22) attached to said containers by a plurality of valves (21, 23) **characterized in that** it also comprises:
- a microbial sensor (5) for analyses in one of the containers (14), said container being called deposit (14), designed to hold a water sample (20) and chemical components by the combining operation of the pump (22) and valves (21, 23), connected to the containers (17,18,19) which hold the chemical components,
- a physicochemical sensor (2) configured to detect different parameters at a certain depth, in relation to the surface of the body of water;
- control and acquisition means (7) configured to selectively control valve (21, 23) and pump (22) operation, and gather data provided by the sensors in a storage means,
- control and acquisition means (7) configured to control a communication module for data communication.

2. Beacon according to claim 1, where the physicochemical sensor (2) is configured to detect at least one of the following parameters: temperature, conductivity, pH, salinity, dissolved oxygen, turbidity, chlorophyll, blue-green algae, or a combination thereof.

3. Beacon according to claim 1 or 2, where the microbial sensor (5) is configured to quantify at least one of the following organisms: Escherichia Coli, Coliforms and/or Enterococcus.

4. Beacon according to any of the preceding claims, where the control and acquisition means are also configured to control a second pump (12), said pump (12) being connected to the deposit (14) so the content of the pump is extracted and poured into a disposal container (13) according to a signal emitted by an overflow sensor.

5. Beacon according to claim 4, where the control and acquisition means are configured to control the first pump (22) and the electrovalves (21) for the washing and emptying of the deposit (14).

6. Beacon according to any of the preceding claims, where the control means (7) comprise a controller configured to receive analogical signals from pressure, temperature and fluorescence sensors.

7. Beacon according to claim 6, where said controller is also configured to control temperature by means of a peltier cooling controller.

8. Beacon according to any of the preceding claims, **characterized in that** the communication module is configured to implement a satellite tracking system.

9. Beacon according to any of the preceding claims, **characterized in that** the communication module is configured to transmit and receive signals under at least one of the following technologies:
- GSM
- GPRS
- radio
or a combination thereof.

10. Beacon according to any of the preceding claims, **characterized in that** it also comprises a wave sensor (3).

11. Beacon according to any of the preceding claims, **characterized in that** it also comprises a casing (1) and a plurality of solar panels (8) located in the part of said casing which is not submerged (1), and connected to batteries (4) which supply energy to the electric devices.
